# EUROPEAN PATENT SPECIFICATION

(11) **EP 0 517 766 B1**
(45) Date of publication and mention of the grant of the patent: **13.09.1995**
(21) Application number: 91905105.2
(22) Date of filing: 01.03.1991
(51) Int. Cl.: C12P 19/04, C12P 19/12

(54) **BIOCHEMICAL PROCESS TO PRODUCE OLIGOSACCHARIDES**
BIOCHEMISCHES VERFAHREN ZUR HERSTELLUNG VON OLIGOSACCHARIDEN
PROCEDE BIOCHIMIQUE DE PRODUCTION D'OLIGOSACCHARIDES

(30) Priority: 02.03.1990 SE 9000758
(43) Date of publication of application: 16.12.1992
(73) Proprietor: PROCUR AKTIEBOLAG, 222 53 Lund (SE)
(72) Inventor: NILSSON, Kurt, S-222 53 Lund (SE)
(74) Representative: Wiklund, Ingrid Helena
(86) International application number: SE9100165
(87) International publication number: WO9113164

(56) References cited:
- EP-A- 266 177
- EP-A- 0 301 522
- WO-A-87/05936
- WO-A-89/09275
- WO-A-91/02806
- US-A- 4 788 145
- US-A- 4 895 801
- US-A- 4 957 860
- PATENT ABSTRACTS OF JAPAN, vol. 12, no. 326 (C525)/

## Description

The present invention relates to a method for enzymatic synthesis of an oligosaccharide compound, which either consists of or is a fragment or an analog of the carbohydrate part in a glycoconjugate.

It has been found that the oligosaccharide part of various glycoconjugates (especially glycolipids and glycoproteins) have a number of important functions in vivo (Biology of Carbohydrates, vol. 2, Ginsburg et al., Wiley, New York, 1984; The Glycoconjugates, vol. I-V, Academic Press, New York; S. Hakomori, Ann. Rev. Biochem., vol. 50, pp. 733-64); Feizi, Nature, pp. 314, 1985; S. Hakomori, Chemistry and Physics of Lipids, vol 42, pp. 209-33). Among other things it was found that
- the carbohydrate structures are important for the stability, activity, localisation, immunogenicity and degradation of glycoproteins;
- carbohydrates are antigenic determinants (for example blood group antigens);
- carbohydrates function as receptors when bound to cell surfaces for pathogens, proteins, hormones, toxins and during cell-cell interactions;
- carbohydrates are important for oncogenesis, since specific oligosaccharides have been found to be cancer-associated antigenic determinants;
- frequently, only a smaller sequence (di- or trisaccharide) of the carbohydrate part of the glycoconjugate is required for full biological activity (e.g. receptor activity).

Universities and Industry are at present working intensely on developing the use of biologically active oligosaccharides within a number of different fields, such as
- novel diagnostics and blood typing reagents
- highly specific materials for affinity chromatography
- cell specific agglutination reagents
- targetting of drugs
- monoclonal antibodies, specific against e.g. cancer-associated structures
- therapy
Besides the above mentioned areas, a considerable future market is envisaged for fine chemicals based on biologically active carbohydrates.

The organic chemical techniques used today for synthesis of these carbohydrate structures require an extensive protective group chemistry with many steps of synthesis and expensive catalysts. Low total yields are obtained in these complicated reaction schemes and the technique is not favorable, especially for larger scale work.

Enzymes are nature's own catalysts with many attractive properties, such as high stereo-, regio-, and substrate selectivity as well as high catalytic activity under mild conditions. Today, great hopes are therefore placed in being able to utilise enzymes for large-scale selective synthesis of oligosaccharides with fewer reaction steps and as a consequence, higher total yields than obtained with organic chemical methodology.

Both hydrolases (glycosidases, EC 3.2) and glycosyltransferases (EC 2.4) can be used for synthesis (glycosidases: see Nisizawa et al, in "The Carbohydrates, Chemistry and Biochemistry", 2nd ed., vol IIA, pp. 242-290, Academic Press, New York, 1970). With glycosidases reversed hydrolysis (equilibrium reaction) or transglycosylation (kinetic reaction) are often used to obtain synthesis (see e.g. K.G.I. Nilsson, Carbohydr. Res., vol. 167, pp. 95-103, 1987, Trends in Biotechnology., vol. 6, pp. 256-264, 1988). With transferases, a nucleotide sugar (UDP-Gal, CMP-Sia, UDP-GalNAc, GDP-Fuc, etc), which is relatively expensive, is used as donor. Moreover, glycosidases are abundant and can often be used directly without purification However, disadvantages with glycosidases are that relatively low yields and wrong product isomers often are obtained. Yields of disaccharides of between 5 and 45 % have been obtained in transglycosylation reactions with glycosidases. In the synthesis of higher oligosaccharides with exoglycosidases, that means when a monosaccharide glycoside or an oligosaccharide is used as a donor and a disaccharide, a higher oligosaccharide or a glycoside thereof is used as acceptor, lower yields have been obtained. This makes the reactions unsuitable for e.g. large-scale synthesis. Equilibrium reactions also give low yields because of the unfavorable equilibrium constants.

WO87/05936 describes the synthesis of a mixture of different regioisomers of dimannose, trimannose and higher oligomannosides, where mannose is used as the only substrate in an equilibrium reaction.

US-A-4 895 801 describes the synthesis of sweetener mixtures or complex oligomer mixtures of Gal-Galₙ-Glc, oligomers of milksugar with undefined linkages.

EP-A-266 177 (Kabushiki Kaisha Yahult Honsha) describes a method for producing oligosaccharides by the reaction between lactose and β-galactosidase from Aspergillus Oryzae.

WO89/09275 (Nilsson, Kurt, G. I.) relates to a method of production of an oligosaccharide compound by using acceptors in lower concentrations.

WO91/02806 (Nilsson, Kurt, G. I.), which is prior art in accordance with Article 54 (3) and(4) EPC, relates to a process for producing an oligosaccharide compound by using glycosidases from a mollusc.

The method according to the present invention allows glycosidase-catalysed synthesis of glycoconjugate oligosaccharide compounds in considerably higher yields than achieved with previous methods. This is achieved according to the invention by using a high concentration of dissolved acceptor substance in transglycosylation reactions so that the reaction mixture (usually solvent, glycosyl donor, glycosyl acceptor, enzyme and buffer salts) is saturated to 30 % or more with the acceptor substance at all conditions of the reaction. The high concentration of acceptor is achieved by for example dissolving more and more of the acceptor substance at a temperature which is close to the boiling point of the solvent (usually buffered water). In this way can, according to the invention, very high acceptor concentrations be used (for example ca 1 M concentration or higher of nitrophenyl glycosides and 2 M or higher of methyl glycosides), which are stable (the acceptor does not precipitate, crystallise from the solution) at the temperature which is used for the transglycosylation reaction, and which are substantially higher than what have been used in previous transglycosylation methods (e.g. 0.15 M nitrophenyl glycoside and 0.6 M of methyl glycoside) for the synthesis of glycoconjugate oligosaccharide structures. The yield of transglycosylation products calculated on donor glycoside (cf. the reaction scheme for transglycosylation reactions in the articles referred to on p. 2) is very high with the method according to the invention, often between 40 and 80 % for synthesis of disaccharides. According to the invention, disaccharides and higher oligosaccharides can also be dissolved in considerably higher concentrations than what have been used in previous transglycosylation reactions and thus, according to the invention exoglycosidases can be used for preparative synthesis of tri-, tetra- and higher oligosaccharides in satisfactory yields.

The substrates are selected with regard to the oligosaccharide which is to be synthesised, and are often commercially available or can be synthesised by organic or enzymatic methods and therefore do not restrict the use of the invention. The donor and acceptor substrates which are used according to the invention are of the same type that have been used in previous transglycosylation reactions (see for example the articles in Carbohydrate Res. vol. 167 and in Trends in Biotechnology vol. 6, referred to in p. 3 above).

As examples of acceptor substances which can be used with the method according to the invention can be mentioned mono-, di-, or oligosaccharides (or glycosides thereof) in which the carbohydrate part contains one or more of the monosaccharides: D-galactose, D-mannose, N-acetyl-neuraminic acid, N-acetyl-D-galactosamin, N-acetyl-D-glucosamine and L-fucose, or an analog of these. When the acceptor substance is a glycoside, the aglycone can be a glycosidically bound flourine or an O-, N-, C-, or S-glycosidically bound (α- or β-configuration) aliphatic or aromatic compound (as for example methyl, ethyl, 2-bromoethyl, (CH₂)ₙCOOMe, n>1, allyl, benzyl, trimethylsilylethyl, amino acid, a derivative thereof, peptide, a derivative thereof, nitrophenyl, etc). Examples of disaccharides which can be used as acceptors are lactose, GlcNAcβ1-3Gal, Galα1-4Gal, Manα1-2Man, GalNAcβ1-3Gal and O-, N-, C-, or S-glycosides (α- or β-configuration) of these as mentioned above.

The donor substrates which can be used with the method according to the invention are the same as those employed in previous methods involving enzymatic transglycosylations (see references on p. 2 above) and thus do not limit the scope of the invention.

As examples of donor substances that can be used with the method according to the invention may be mentioned monosaccharide glycosides and di- or oligosaccharides (or glycosides thereof) in which the carbohydrate part contains one or more of the monosaccharides D-galactose, D-mannose, N-acetyl-neuraminic acid, N-acetyl-D-galactosamin, N-acetyl-D-glucosamine and L-fucose. As examples of suitable glycosyl donors may be mentioned α- or β-bound nitrophenyl-, methyl-or allyl-glycosides of the monosaccharides above, lactose, dimannose and raffinose. Oligosaccharides and glycosides of disaccharides or oligosaccharides which are substrates for endoglycosidases are also suitable as donor substrates.

The concentration of the glycosyl donor in the reaction mixture is selected with regard to the oligosaccharide which is to be synthesised and also with regard to the properties of the enzyme and therefore do not restrict the use of the invention. In general, addition of the donor in smaller portions may be advantageous in order to minimise the risk that the donor also acts as an acceptor (unless this is desired; see the example below with Galα-OPhNO₂ as donor).

The enzymes are selected primarily with regard to which oligosaccharide is to be synthesised. The enzyme may be used in situ or after partial or complete purification from their natural environment. The enzyme may be used in soluble form or immobilised to a solid support by e.g. adsorption, encapsulation, chelation, precipitation or covalent binding.

Examples of α- and β-glycosidases which may be used according to the invention are D-mannosidases, D-galactosidases, L-fucosidases, N-acetyl-D-galactosaminidases, hexosaminidases and other glycosidases of EC group 3.2 (Enzyme Nomenclature, Academic Press, 1984). Both endo- and exoglycosidases may be used with the method according to the invention. Glycosidases obtained from a mollusc may not be used according to the present invention.

In addition to the glycosidase which is employed to catalyse the transglycosylation reaction, another type of enzyme can be added to the reaction mixture to degrade or convert the hydrolysis product of the donor substance (for example, mannose is formed when a mannose glycoside is used as donor). In this way can any inhibition of the glycosidase-catalysed reaction be minimised. In this case, an enzyme that selectively converts the hydrolysis product to a product which inhibit the glycosidase-reaction to a minimal extent, is preferentially used.

The enzyme is added after the substrates and at the desired temperature. The reaction temperature and pH are chosen with regard to the temperature sensitivity and pH-dependence of the enzyme and do not limit the method of the invention. A higher temperature than room temperature decreases the risk of precipitation of the acceptor substance and increases the reaction rate. It is therefore often suitable to carry out the reaction at the highest temperature possible which does not denature and inactivate the enzyme.

Precipitation of the excess of acceptor substance using for example cooling of the reaction mixture and/or addition of organic solvent, can be used in the isolation of the transglycosylation product.

The synthesis method according to the invention is generally applicable to the synthesis of oligosaccharide sequences included in glycoconjugates (see examples of structures given in references on page 1 above). Of special interest are the minutest fragments of these structures, which are sufficient to transfer biological activity and the choice of donor and acceptor in the equilibrium or transglycosylation reaction is determined by this.

Examples of interesting structures are blood group determinants, cancer-associated oligosaccharide structures and structures with biological receptor activity (see references on page 1 above).

An example of how the invention may be used in actual practice is described in the following Example, which, however, is in no way intended to restrict the scope of the invention (abbreviations according to IUPAC-IUB's recommendations, J. Biol. Chem., vol. 257, pp. 3347-3354, 1982).

### EXAMPLE

Synthesis of Galα1-3Galα-OPhNO₂-p: Galα-OPhNO₂-p (300 mg) was dissolved in 0.04 M sodium phosphate buffer, pH 6.5 (0.85 ml) and N,N-dimethylformamide (0.05 ml) at 95-100 °C. After cooling to 50 °C, 100 microliter of α-galactosidase from green coffee beans (Sigma, St. Louis; 10 U) was added. HPLC (C₁₈-silica; 20 % acetonitrile in water as eluent) showed that about 200 mg of Galα-OPhNO₂ -p was reacted after 5 h and that about 65 % of this had been converted to the desired α1-3 bound digalactoside. Initially, the yield was close to 90 %. The product was isolated with column chromatography (Sephadex G10®).

## Claims

1. A method to produce an oligosaccharide compound, which either consists of or is a fragment or an analog of the carbohydrate part in a glycoconjugate by reacting a donor substance with an oligosaccharide or a monosaccharide glycoside, with an acceptor substance, which is a monosaccharide or an oligosaccharide, or a glycoside or a saccharide analog in a transglycosylation reaction, **characterised** in that at least one glycosidase (EC 3.2), but not a glycosidase obtained from a mollusc, is used and that the concentration of the acceptor substance is very high and is such that the reaction mixture is saturated 30 % or more with regard to the acceptor substance at the start and subsequent conditions for the reaction, and that the oligosaccharide compound is separated from the reaction mixture.

2. A method as claimed in claim 1, **characterised** in that the carbohydrate part in the donor- and in the acceptor substance contains one or more of the monosaccharides D-galactose, D-mannose, N-acetylneuraminic acid, N-acetyl-D-galactosamine, N-acetyl-D-glucosamine and L-fucose, or is an analog of these.

3. A method as claimed in claim 1, **characterised** in that the acceptor substance is a glycoside in which the aglycone is glycosidically bound flourine or is an O-, N-, C-, or S-glycosidically bound alifatic or aromatic compound.

4. A method as claimed in one or more of the previous claims, **characterised** in that the enzyme (the glycosidase) is used in situ or after it has been isolated completely or partly from its natural biological environment.

5. A method as claimed in one or more of the previous claims, **characterised** in that the enzyme is immobilised via precipitation, adsorption, enclosure, chelation, or covalent bonding, to a polymeric substance or derivative thereof which is insoluble in protic or aprotic solvents.

6. A method as claimed in one or more of the previous claims, **characterised** in that the polymeric substance consists of a polysaccharide (cellulose, agarose, etc), a plastic (polyacrylamide, polyvinylalcohol, polystyrene, etc), a copolymer, a polysaccharide-plastic, a silicate, or a glass, and which has been activated and contains reactive groups as for example cyanate, organic sulphonates, aldehyde, diazonium, epoxi, divinylsulphone, or triazin groups.

7. A method as claimed in one or more of the previous claims, **characterised** in that one of the substances Fucα1-6Galβ-OMe, GalNAcα1-3Galα-OMe, GalNAcβ1-3Galβ-OMe, GlcNAcβ1-6Galα-OMe, GlcNAcβ1-6Manα-OMe or GlcNAcβ1-3Galβ-OMe is synthesised and isolated.

8. A method as claimed in one or more of claims 1-7, **characterised** in that at least one other type of enzyme than a glycosidase is used in the reaction to convert the hydrolysis product.

## Patentansprüche

1. Verfahren zum Herstellen einer Oligosaccharid-Verbindung, welche entweder aus dem Kohlenhydrat-Anteil in einem Glycokonjugat besteht oder ein Fragment oder ein Analogon davon ist, durch Umsetzen einer Donorsubstanz mit einem Oligosaccharid- oder einem Monosaccharid-Glycosid, mit einer Akzeptorsubstanz, welche ein Monosaccharid oder ein Oligosaccharid oder ein Glycosid- oder ein Saccharid-Analogon ist, in einer Transglycosylierungsreaktion, **dadurch gekennzeichnet**, daß mindestens eine Glycosidase (EC 3.2), aber nicht eine Glycosidase, die aus einer Molluske erhalten wurde, verwendet wird, und daß die Konzentration der Akzeptorsubstanz sehr hoch ist und so ist, daß das Reaktionsgemisch bezüglich der Akzeptorsubstanz bei Beginn und nachfolgenden Reaktionsbedingungen zu 30% oder mehr gesättigt ist, und daß die Oligosaccharid-Verbindung von dem Reaktionsgemisch abgetrennt wird.

2. Verfahren nach Anspruch 1, **dadurch gekennzeichnet**, daß der Kohlenhydrat-Anteil in der Donor- und in der Akzeptorsubstanz eines oder mehrere der Monosaccharide D-Galactose, D-Mannose, N-Acetyl-neuraminsäure, N-Acetyl-D-galactosamin, N-Acetyl-D-glucosamin und L-Fucose enthält oder ein Analogon von diesen ist.

3. Verfahren nach Anspruch 1, **dadurch gekennzeichnet**, daß die Akzeptorsubstanz ein Glycosid ist, in welchem das Aglycon glycosidisch gebundenes Fluor oder eine O-, H-, C- oder S-glycosidisch gebundene aliphatische oder aromatische Verbindung ist.

4. Verfahren nach einem oder mehreren der vorangehenden Ansprüche, **dadurch gekennzeichnet**, daß das Enzym (die Glycosidase) in situ oder nach vollständiger oder teilweiser Isolierung aus seiner natürlichen biologischen Umgebung verwendet wird.

5. Verfahren nach einem oder mehreren der vorangehenden Ansprüche, **dadurch gekennzeichnet**, daß das Enzym durch Fällung, Adsorption, Einschluß, Chelatbildung oder kovalente Bindung an einer polymeren Substanz oder einem Derivat davon, welche(s) in protischen oder aprotischen Lösungsmitteln unlöslich ist, immobilisiert ist.

6. Verfahren nach einem oder mehreren der vorangehenden Ansprüche, **dadurch gekennzeichnet**, daß die polymere Substanz aus einem Polysaccharid (Cellulose, Agarose usw.), einem Kunststoff (Polyacrylamid, Polyvinylalkohol, Polystyrol usw.), einem Copolymer, einem Polysaccharid-Kunststoff, einem Silicat oder einem Glas besteht und aktiviert worden ist und reaktive Gruppen wie z.B. Cyanat-, organische Sulfonat-, Aldehyd-, Diazonium-, Epoxy-, Divinylsulfon- oder Triazin-Gruppen enthält.

7. Verfahren nach einem oder mehreren der vorangehenden Ansprüche, **dadurch gekennzeichnet**, daß eine der Substanzen Fucα1-6Galβ-OMe, GalNAcα1-3Galα-OMe, GalNAcβ1-3Galβ-OMe, GlcNAcβ1-6Galα-OMe, GlcNAcβ1-6Manα-OMe oder GlcNAcβ1-3Galβ-OMe synthetisiert und isoliert wird.

8. Verfahren nach einem oder mehreren der Ansprüche 1-7, **dadurch gekennzeichnet**, daß mindestens eine weitere Art von Enzym, welches keine Glycosidase ist, in der Reaktion verwendet wird, um das Hydrolyseprodukt umzuwandeln.

## Revendications

1. Procédé de production d'un composé oligosaccharidique, qui est constitué de ou qui est un fragment ou un analogue de la partie glucidique d'un glycoconjugué, en faisant réagir une substance donneuse avec un oligosaccharide ou un glycoside monosaccharidique, avec une substance acceptrice, qui est un monosaccharide ou un oligosaccharide ou un glycoside ou un analogue de saccharide dans une réaction de transglycosylation, caractérisé en ce qu'on utilise au moins une glycosidase (EC 3.2), mais pas une glycosidase obtenue à partir d'un mollusque, et en ce que la concentration en substance acceptrice est très élevée et est telle que le mélange réactionnel est saturé à 30% ou plus par la substance acceptrice au départ et dans les conditions ultérieures de la réaction, et en ce que le composé oligosaccharidique est séparé du mélange réactionnel.

2. Procédé tel que revendiqué dans la revendication 1, caractérisé en ce que la partie glucidique de la substance donneuse et celle de la substance acceptrice contiennent un ou plusieurs des monosaccharides D-galactose, D-mannose, acide N-acétylneuraminique, N-acétyl-D-galactosamine, N-acétyl-D-glucosamine et L-fucose, ou sont des analogues de ceux-ci.

3. Procédé tel que revendiqué dans la revendication 1, caractérisé en ce que la substance acceptrice est un glycoside dans lequel l'aglycone est un fluor lié par une liaison glycosidique ou un composé aliphatique ou aromatique lié par une liaison O-, N, C- ou S-glycosidique.

4. Procédé tel que revendiqué dans l'une ou plusieurs des revendications précédentes, caractérisé en ce que l'enzyme (la glycosidase) est utilisée in situ ou après avoir été isolée en totalité ou en partie à partir de son environnement biologique naturel.

5. Procédé tel que revendiqué dans l'une ou plusieurs des revendications précédentes, caractérisé en ce que l'enzyme est immobilisée par précipitation adsorption, enfermement, chélation, ou liaison covalente, à une substance polymère ou à un dérivé de celle-ci qui est insoluble dans les solvants protiques ou aprotiques.

6. Procédé tel que revendiqué dans l'une ou plusieurs des revendications précédentes, caractérisé en ce que la substance polymère est constituee d'un polysaccharide, (cellulose, agarose, etc), d'un plastique (polyacrylamide, polyvinylalcool, polystyrène, etc), d'un copolymère d'un polysaccharide-plastique, d'un silicate, ou d'un verre, et qu'elle a été activée et contient des groupes réactifs tels que par exemples des groupes cyanate, sulphonates organiques, aldéhyde, diazonium, époxy, divinylsulphone, ou triazine.

7. Procédé tel que revendiqué dans l'une ou plusieurs des revendications précédentes, caractérisé en ce qu'une des substances Fucα1-6Galβ-OMe, GalNacα1-3Galα-OMe, GalNAcβ1-3Galβ-OMe, GlcNAcβ1-6Galα-OMe, GlcNAcβ1-6Manα-OMe ou GlcNAcβ1-3Galβ-OMe est synthétisée et isolée.

8. Procédé tel que revendiqué dans l'une ou plusieurs des revendications 1 à 7, caractérisé en ce qu'au moins une enzyme d'un autre type qu'une glycosidase est utilisée dans la réaction pour convertir le produit d'hydrolyse.
